(19) Europäisches Patentamt
European Patent Office
Office européen des brevets

(11) EP 2 161 342 A2

(12) **DEMANDE DE BREVET EUROPEEN**

(43) Date de publication:
**10.03.2010 Bulletin 2010/10**

(51) Int Cl.:
***C12Q 1/64*** *(2006.01)* ***G06F 19/00*** *(2006.01)*

(21) Numéro de dépôt: **09290582.7**

(22) Date de dépôt: **23.07.2009**

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **01.08.2008 FR 0804436**

(71) Demandeur: **IFP**
**92852 Rueil-Malmaison Cédex (FR)**

(72) Inventeurs:
- **Haeseler, Frank**
  **92250 La Garenne Colombes (FR)**
- **Behar, Françoise**
  **75007 Paris (FR)**
- **Chenet, Pierre-Yves**
  **77450 Condé Sainte Libiaire (FR)**

(54) **Modèle de bassin pour prédire les changements de composition chimique d'huile d'une réservoir suite à une biodégradation**

(57) - Méthode mise en oeuvre par ordinateur pour déterminer une composition d'hydrocarbures présents dans un bassin sédimentaire suite à une biodégradation.
- On estime les vitesses de déplacement des hydrocarbures et les vitesses de déplacement de l'eau présente dans le milieu poreux, depuis un instant de genèse dans une roche mère jusqu'à une accumulation éventuelle dans une roche réservoir. Puis, on modélise la biodégradation dans l'ensemble du milieu poreux en fonction du temps, depuis la genèse dans la roche mère, en considérant que la composition d'hydrocarbures varie proportionnellement aux vitesses de déplacement
- Application notamment à l'exploitation de gisements pétroliers.

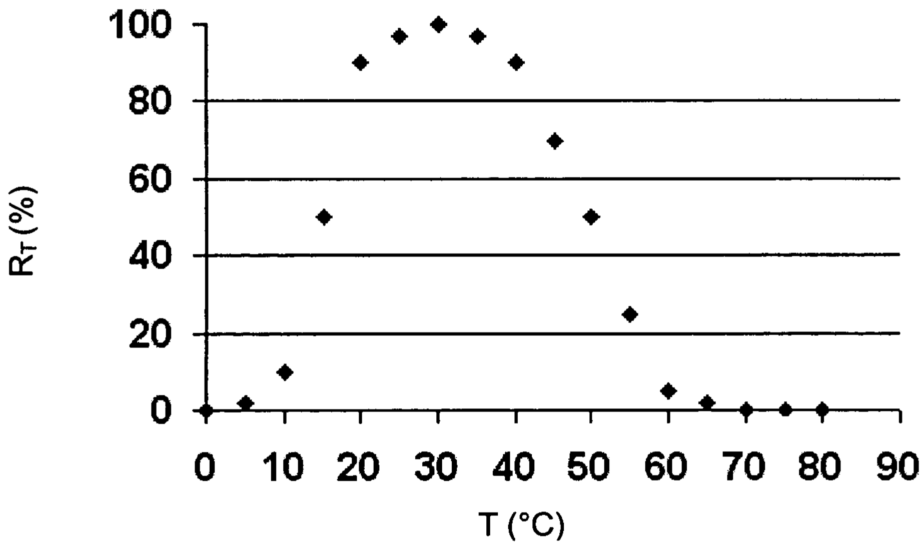


Fig. 4

EP 2 161 342 A2

## Description

La présente invention concerne le domaine de l'industrie pétrolière. Elle concerne une méthode pour évaluer la biodégradation d'hydrocarbures présents dans une structure géologique, telle qu'un gisement pétrolier, suite à l'action d'une population bactérienne.

La méthode selon l'invention fournit un outil d'évaluation très utile notamment aux géologues soucieux d'orienter les investigations vers des zones à risque. En particulier, on peut l'utiliser dans le domaine technique de la modélisation de bassin pour prédire la quantité et la qualité de l'huile que l'on peut s'attendre à trouver en relation avec l'altération biologique qui peut advenir dans de tels environnements.

Un des problèmes couramment rencontrés lors de la définition de l'intérêt d'un objectif pétrolier, c'est-à-dire un piège à hydrocarbures non foré, situé à une relativement faible température (habituellement à moins de 80°C) est l'évaluation du risque de "biodégradation".

En effet il est couramment reconnu que la biodégradation, définie comme la destruction sélective d'une partie des molécules composant un brut pétrolier par des bactéries, peut se développer jusqu'à des températures pouvant atteindre 70 à 80°C. De telles températures sont courantes en particulier dans les sédiments marins qui sont des zones où la recherche pétrolière est la plus active actuellement.

Cette biodégradation a pour effet de modifier la qualité de l'huile. Cette qualité est mesurée par la viscosité et le degré API qui est inversement proportionnel à la densité. Elle est fonction de la composition de l'huile (l'échelle de degrés API est entre 0 et 40). Une forte teneur en hydrocarbures légers, augmente le degré API, alors qu'une forte proportion de résines et d'asphaltènes (NSO) fait décroître le degré API. Au cours de l'histoire géologique d'un fluide, la maturation thermique tend à alléger les huiles de réservoir, donc tend à augmenter le degré API, tandis que l'altération microbienne tend à alourdir l'huile, et donc à décroître son degré API (Conan et al. 1979 Advances in Organic Geochemistry, Pergamon Press Oxford 1-17 et Peters et Moldowan, 1993, The biomarker guide, Prentice Hall).

Un autre problème induit par la biodégradation des hydrocarbures est la production de gaz (méthane et gaz acides : $CO_2$ et $H_2S$). Certains de ces gaz ont une valeur économique (c'est le cas du méthane) et d'autres réduisent la valeur économique du réservoir ($H_2S$).

La biodégradation est un phénomène biologique qui peut affecter la quantité et la composition chimique des huiles de réservoir. Les bactéries, responsables de ces réactions, sont présentes dans le milieu poreux et vivent dans l'eau qui circule dans le milieu poreux. Ces bactéries utilisent les hydrocarbures comme source de carbone et d'énergie pour assurer leur développement et leur maintien. En faisant cela, d'une part elle dégradent sélectivement certains hydrocarbures et donc modifient à la fois la quantité et la composition de l'huile en place et d'autre part elles génèrent des métabolites comme des espèces gazeuses ($CH_4$, $H_2S$ et $CO_2$) et de nouvelles structures chimiques comme les acides carboxyliques. Néanmoins, les acides générés sont à leur tour dégradés et ne représentent en fait qu'une faible quantité dans les huiles biodégradées résiduelles. Pour faire vivre les bactéries, il faut également apporter au système des ingrédients comme les accepteurs d'électrons et les nutriments. Les réactions biologiques ont besoin de nutriments comme le phosphore et l'azote ainsi que des métaux, éléments indispensables à la synthèse des molécules constitutives des bactéries. Leur absence signifie l'arrêt de la croissance bactérienne.

Le processus de biodégradation peut être décrit de la façon suivante. Les microorganismes présents dans les milieux poreux utilisent la source d'énergie et de carbone que constituent certaines familles d'hydrocarbures présentes dans les huiles de réservoir avec deux objectifs :

- se développer et produire le plus possible de biomasse tant que l'azote et le phosphore ne sont pas épuisés et que les éléments en trace comme les métaux ne sont pas des facteurs limitants

- se maintenir, c'est à dire utiliser l'énergie disponible à travers des réactions de dégradation qui ne génèrent pas de biomasse supplémentaire. Durant ces réactions, les hydrocarbures sont effectivement dégradés et des métabolites sont générés mais aucun accroissement de biomasse n'est observé;

Ainsi, dans l'industrie pétrolière, il est très important de connaître le rôle de la biodégradation pour connaître la qualité et la quantité d'huile attendue dans un bassin sédimentaire. En effet, la biodégradation représente un risque majeur pour les compagnies pétrolières dont les forages, par exemple en mer profonde, représentent un investissement financier important. Toute méthode permettant de réduire ce risque est donc d'un intérêt majeur pour ces compagnies. Pour y parvenir, il faut disposer d'une méthode permettant d'estimer l'effet de la biodégradation sur la quantité et la qualité de l'huile.

### Présentation de l'art antérieur

Certains auteurs ont pu estimer des cinétiques de dégradation en corrélant des temps de séjour de l'huile dans

le sous-sol avec le niveau de biodégradation (Larter et al 2003 Organic Geochemistry 4, 6001-613, Behar et al, 2006, Organic Geochemistry 37, 1042-1051 et de Barros Penteado et al, 2007, Organic Geochemistry 38, 1197-1211). Il s'agit là essentiellement d'un travail descriptif, visant à constater la biodégradation et ses conséquences, ainsi qu'à déterminer pour certains bassins des vitesses spécifiques de biodégradation. En particulier, les travaux de Larter permettent de prédire un taux de biodégradation sans tenir compte de la composition de l'huile, ce taux étant principalement appliqué aux hydrocarbures saturés. Par ailleurs, le moteur de la biodégradation est la diffusion des hydrocarbures au niveau de la zone de contact eau/huile dans la mesure ou c'est cette zone de contact qui fournit la source des accepteurs d'électrons.

Pour estimer de façon prédictive le niveau de biodégradation des huiles dans un bassin, on connaît une méthode décrite dans le brevet EP 1.436.412. Cette méthodologie est basée sur une analyse statistique du nombre de bactéries présentes dans le sous-sol en fonction de la profondeur. Par ailleurs, le résultat de cette approche n'est pas compositionnel.

De façon générale, les méthodes connues visent soit à décrire la biodégradation et non à la prédire, soit à prédire la biodégradation en prenant en compte le plan de contact eau/huile pour contrôler les vitesses de biodégradation. Ces types de méthodes ne tiennent pas compte des facteurs limitants l'activité des microorganismes : les accepteurs d'électrons. Ainsi, ces méthodes ne sont pas réellement prédictives et nécessitent un calage avec des données de puits.

Ainsi, l'objet de l'invention est un outil logiciel pour modéliser l'évolution compositionnelle d'hydrocarbures présents dans un milieu poreux suite à une biodégradation.

## La méthode selon l'invention

L'invention concerne une méthode mise en oeuvre par ordinateur pour déterminer une composition d'hydrocarbures présents dans un milieu poreux d'un bassin sédimentaire, dans laquelle on modélise une biodégradation subie par lesdits hydrocarbures. La méthode comporte les étapes suivantes :

- on estime des vitesses de déplacement des hydrocarbures et des vitesses de déplacement de l'eau présente dans ledit milieu poreux, depuis un instant de genèse dans une roche mère jusqu'à une accumulation éventuelle dans une roche réservoir, et
- on modélise la biodégradation dans l'ensemble du milieu poreux en fonction du temps, depuis la genèse dans la roche mère, en considérant que la composition d'hydrocarbures varie proportionnellement aux vitesses de déplacement.

Selon un mode de réalisation, la modélisation comporte les étapes suivantes :

- on discrétise ledit milieu poreux dudit bassin sédimentaire en un ensemble de mailles ;

- on définit un schéma compositionnel de biodégradation à partir d'au moins les classes chimiques suivantes : $CO_2$, $H_2S$, $C_1$, $C_2$-$C_4$, $C_6$-$C_{14}$ saturés n et $C_6$-$C_{14}$ saturés iso, $C_6$-$C_{14}$ saturés cyclo, $C_6$-$C_{14}$ aromatiques, $C_{14+}$ saturés n, $C_{14+}$ saturés iso, $C_{14+}$ saturés cyclo, $C_{14+}$ aromatiques, NSOs ;

- on détermine, à l'instant de genèse des hydrocarbures, une quantité $C_i$ de chacune desdites classes chimiques contenues dans lesdits d'hydrocarbures ;

- on définit un pas de temps $\Delta t$, discrétisant le temps à partir de la genèse des hydrocarbures, et pour chaque maille et chaque pas de temps $\Delta t$ :

- on estime lesdites vitesses de déplacement en déterminant une variation de saturation moyenne en hydrocarbure $\Delta Sat_{HC}$ et une saturation en eau disponible pour la réaction de biodégradation $Sat_{WBIO}$;

- on détermine une température T du milieu poreux ;

- on détermine des vitesses de biodégradations $V_i$ pour chacune desdites classes chimiques, en fonction de la concentration potentielle des classes chimiques dans l'eau, de leur biodégradabilité intrinsèque, de leur réactivité par rapport auxdits accepteurs d'électrons, de la température du milieu, et du temps de séjour desdits hydrocarbures au sein du milieu ; et

- on détermine une variation de concentration $\Delta C_i$ d'une classe chimique i au cours de l'intervalle de temps $\Delta t$, à partir desdites quantités d'hydrocarbures, de $\Delta Sat_{HC}$, de $Sat_{WBIO}$, et de T, et desdites vitesses de biodégradation.

Selon l'invention, on peut déterminer la variation de concentration $\Delta Ci$ au moyen de la formule suivante :

$$\Delta C_i = - V_i(\Delta t, T)^* \Delta Sat_{HC}^* Sat_{WBIO}^* Ci(t_n)^* \Delta t * I_{STERIL},$$

avec :

$$\Delta t = t_{n+1} - t_n.$$

$I_{STERIL}$ = 1 si la température T n'est jamais supérieure à une température de pasteurisation,

$I_{STERIL}$ = 0 sinon.

On peut définir les vitesses de réactions par un produit d'un premier terme ($R_t$) qui permet de prendre en compte un effet du temps de séjour dans le milieu poreux, un second terme ($R_T$) pour prendre en compte un effet de la température, et un troisième terme ($K_C$) pour prendre en compte l'accessibilité et la biodégradabilité intrinsèque de chacune des classes chimiques.

Le premier terme $R_t$ peut représenter un rendement de biodégradation et peut être déterminé au moyen d'une première courbe d'évolution du rendement $R_t$ en fonction du temps de séjour dans le milieu. Cette courbe peut être une fonction exponentielle du temps,
où le rendement $R_t$ est supérieur à 95 % au-delà d'environ 2000 ans.

Le second terme $R_T$ peut représenter un rendement de biodégradation et peut être déterminé au moyen d'une courbe gaussienne d'évolution du rendement $R_T$ en fonction de la température dans le milieu. Cette courbe gaussienne est avantageusement centrée sur une température d'environ 30°C et s'annule pour 0°C et environ 70°C.

Le troisième terme ($V_C$) peut être déterminé en considérant que la vitesse de biodégradabilité intrinsèque des $C_6$-$C_{14}$ saturés est plus grande que celles des autres classes, et en considérant que la vitesse d'accessibilité maximale des $C_6$-$C_{14}$ aromatiques est plus grande que celles des autres classes.
Ainsi, les vitesses de réactions peuvent être définies par :

$$V\ C_{14-}sat = 0.4xV_{max}$$

$$V\ C_{14-}aro = 0.3xV_{max}$$

$$V\ C_{14+}n = 0.2xV_{max}$$

$$V\ C_{14+}iso = 0.18xV_{max}$$

$$V\ C_{14+}cyclanes = 0.08xV_{max}$$

$$V\ C_{14+}aro = 0.05xV_{max}$$

$$V_{NSO} = 0$$

où $V_{max}$ est vitesse maximale de biodégradation définie par le produit de la vitesse maximale de biodégradabilité intrinsèque par la vitesse maximale d'accessibilité.

Selon un mode de réalisation, on détermine également une quantité de gaz acides produit lors de la biodégradation, pour déterminer des conditions d'exploitation d'une roche réservoir dudit bassin.

Enfin, selon un autre mode de réalisation, on détermine également une quantité de méthane produit lors de la biodégradation, pour déterminer des conditions d'exploitation d'une roche réservoir dudit bassin.

## Présentation succincte des figures

D'autres caractéristiques et avantages de la méthode selon l'invention, apparaîtront à la lecture de la description ci-après d'exemples non limitatifs de réalisations, en se référant aux figures annexées et décrites ci-après.

La figure 1 montre un exemple de non accessibilité de l'huile aux micro organismes du milieu aqueux.

La figure 2 illustre un exemple d'évolution de la composition chimique des huiles de réservoir au cours d'une altération croissante par la biodégradation.

La figure 3 illustre l'évolution, en fonction du temps t, du terme $R_t$, terme permettant de prendre en compte dans la vitesse globale de biodégradation l'effet du temps de séjour dans le milieu poreux.

La figure 4 illustre l'évolution, en fonction de la température T, du terme $R_T$, terme permettant de prendre en compte dans la vitesse globale de biodégradation l'effet de la température.

## Description détaillée de la méthode

La méthode selon l'invention permet d'évaluer qualitativement et quantitativement des hydrocarbures présents dans un milieu poreux d'un bassin sédimentaire, après qu'ils aient subi une biodégradation.

Cette méthode est mise en oeuvre par ordinateur, au moyen d'un logiciel appelé par les spécialistes « modèle de bassin » ou « simulateur de bassin ». Un exemple de tels simulateurs, appelé TEMIS$^{Flow}$ ® est décrit dans le document suivant :

> Thibaut M, Sulzer C, Jardin A, Bêche M, 2007, ISBA: A Methodological Project for Petroleum Systems Evaluation in Complex Areas, AAPG Congress, Athens

Ce modèle de bassin permet de modéliser la genèse et le transport des fluides pétroliers depuis une roche mère jusqu'à l'accumulation dans une roche réservoir. Au sein de ce simulateur, la genèse est contrôlée par des équations cinétiques, et le transport est contrôlé par la loi de Darcy.

Selon l'invention, on modélise en plus la biodégradation subie par ces hydrocarbures au cours de leur transport et de leur accumulation. La biodégradation n'est pas modélisée uniquement au niveau de l'interface entre les hydrocarbures et l'eau au sein du réservoir pétrolier, mais dans tout le milieu poreux constituant le bassin et traversé par les hydrocarbures.

Pour ce faire, la méthode permet de modéliser la biodégradation subie par les hydrocarbures dans l'ensemble du milieu poreux, à partir d'une estimation de vitesses de déplacement des hydrocarbures et de l'eau présente dans le milieu poreux, en utilisant la loi de Darcy.

Lorsque le premier fluide hydrocarbure (huile) quitte la roche mère et circule dans le milieu poreux, celui est d'abord rempli d'eau. L'huile peut pénétrer dans un milieu poreux si sa pression $P_{hc}$ arrive à excéder la pression d'eau plus la pression capillaire du milieu, liée à la taille de pore, la mouillabilité et la saturation en hydrocarbures préexistante. On trouve alors les cas de figures suivant :

a- l'huile traverse un milieu poreux mais ne s'y accumule pas. C'est le cas d'une portion de chemin de migration. Dans ce cas, l'huile ne sature que faiblement le milieu (quelques %). Le rapport volume huile/volume eau reste faible. Si les conditions de température le permettent, la biodégradation peut se produire, puisque l'eau est en quantité suffisante, si la vitesse de mouvement des hydrocarbures n'est pas trop élevée par rapport à la vitesse de la réaction de biodégradation.

b- L'huile s'accumule dans un milieu poreux. La biodégradation peut sous certaines conditions se produire et va consommer une certaine quantité d'eau en conditions méthanogènes. En deça d'une certaine saturation en eau, il n'y a plus d'accès à de l'eau, enfermée dans des pores inaccessibles a l'huile pour cause de taille du goulet

d'étranglement (pore throat) trop faible (figure 1). La saturation "$SatIR_{w-bio}$" est la fraction de la saturation irréductible en eau, accessible aux micro organismes. En effet, dans les milieux argileux par exemple, même si les saturations résiduelles en eau peuvent être non négligeables, le contact eau / huile restera très limité du fait des forces capillaires à vaincre ; dans ce cas, la biodégradation sera très vite stoppée, dès que le contact eau - huile aura permis la formation d'un fluide très visqueux ou « tar mat » qui va obstruer la porosité, comme l'illustre la figure 1 qui montre un exemple de non accessibilité de l'huile aux micro organismes du milieu aqueux.

c- Lorsque l'huile se déplace dans le milieu poreux, le volume libéré est replacé par de l'eau. De l'eau étant a nouveau disponible, la biodégradation peut éventuellement reprendre. Elle pourra altérer l'huile résiduelle après échappement de l'huile mobile.

On considère que la biodégradation est proportionnelle à la variation de la saturation dans un volume poreux donné au cours du temps. S'il n'y a pas de variation de la saturation, la vitesse d'entrée des hydrocarbures d'une maille donnée est la même que la vitesse de sortie. Dans ce cas la biodégradation très partielle de l'huile ne se produit qu'au moment de l'invasion du milieu poreux par l'huile, tant qu'il y a de l'eau biodisponible qui sert d'accepteur d'électrons pour la méthanogenèse.

La biodégradation est également fonction du volume d'eau consommé lors de la réaction de méthanogenèse, ce volume ne pouvant excéder une certaine fraction du volume poreux.

A l'échelle du processus élémentaire de mouvement dans les pores, on a pratiquement un milieu soit totalement envahi d'huile, qui se biodégrade par méthanogenèse à concurrence de l'eau disponible au contact, soit totalement envahi d'eau.

A l'échelle d'une maille dans un modèle de bassin, on calcule en pratique une variation de saturation moyenne en hydrocarbure, notée $\Delta Sat_{HC}$. On définit aussi une saturation en eau disponible pour la réaction de biodégradation, notée $SatIR_{WBIO}$. Cette saturation est fonction du type de lithologie. Elle est faible pour des argiles, et est maximum dans des milieux poreux sphériques (type sable).

Le volume relatif d'eau ayant servi à la biodégradation , en cas de réaction complète sera donc : $\Delta Sat_{HC}$* $SatIR_{WBIO}$.

Au cours d'une durée $\Delta t$, ces réactions ont un certain taux d'avancement proportionnel à :

- le rendement de la réaction globale de biodégradation, notée $R_{bio}(T)$, fonction de la température ;
- la vitesse de transformation $V_{ci}$ d'un composant Ci (tenant compte de l'accessibilité et de la biodégradabilité intrinsèque) ;
- le rendement de biodégradation en fonction du temps $R_{bio}(\Delta t)$
- la concentration $C_i$ du composant i (décrit par la genèse pétrolière)
  Enfin, si la température excède une certaine valeur $T_{STERIL}$, il y a « pasteurisation » du milieu (Wilhems A, Larter SR, Head I, Farrimond P, di Primio R, Zwach C (2001) Biodegradation of oil in uplifted basins prevented by deep-burial sterilization. Nature, 411: 1034-1037.), c'est-à-dire que les bactéries sont définitivement tuées et la réaction ne peut plus se produire, même si la température repasse sous le seuil de stérilisation. Un « drapeau » de stérilisation est alors activé au sein du simulateur de bassin, $I_{STERIL}$ = 1, au moment du dépôt du sédiment poreux. Si la température dans une maille est supérieur à $T_{STERIL}$, $I_{STERIL}$ = 0.

La variation de concentration d'un composant $\Delta C_i$ au cours d'un pas de temps $\Delta t$ s'écrit alors entre les temps $t_n$ et $t_{n+1}$ comme une réaction cinétique :

$$\Delta C_i = - K_{ci} * Vbio(T) * Vbio(\Delta t) * \Delta Sat_{HC} * Sat_{WBIO} * Ci\ (t_n) * \Delta t * I_{STERIL}$$

Avec, pour rappel:

$\Delta t$ : intervalle de temps entre des temps $t_n$ et $t_{n+1}$

Ci ($t_n$) : concentration d'un composant chimique i à l'instant $t_n$

| | |
|---|---|
| $\Delta C_i$ : | variation de concentration d'un composant chimique i au cours de l'intervalle de temps $\Delta t$ |
| T : | la température |
| $K_{ci}$ : | vitesse de transformation d'un composant chimique i (accessibilité et biodégradabilité) |
| $R_{bio}(T)$ : | vitesse de la réaction globale de biodégradation |
| $R_{bio}(\Delta t)$ : | taux de biodégradation |
| $\Delta Sat_{HC}$ : | variation de saturation moyenne en hydrocarbure |
| $SatIR_{WBIO}$ : | saturation en eau disponible pour la réaction de biodégradation |

Si le pas de temps est grand (> 50,000 ans), la température optimale (40°C), la variation de saturation maximale (100%), on a alors : $\Delta Ci = -K_{ci} * Sat_{WBIO} * Ci\ (t_n) * \Delta t$

La figure 2 illustre la variation compositionnelle en fonction de l'avancement de la biodégradation, en appliquant une réaction de méthanogènèse sur un fluide de réservoir.

Selon une mise en oeuvre, la méthode peut comporter les étapes suivantes :

1- Discrétisation du milieu poreux en un ensemble de mailles ;

2- Estimation compositionnelle avant biodégradation ;

3- Estimation des vitesses de déplacement des fluides dans le milieu et de la température ;

4- Estimation de vitesses de biodégradations ;

5- Détermination de la variation compositionnelle des hydrocarbures.

<u>1- Estimation compositionnelle avant biodégradation</u>

Ce type d'estimation peut classiquement être réalisée à partir d'un outil, connu des spécialistes, appelé « simulateur de bassin », ou « modèle de bassin ». Un exemple de méthode utilisée par ce type d'outil logiciel est décrit dans la demande de brevet FR 2.906.482. Cette demande décrit une méthode de modélisation du craquage thermique du kérogène et des produits pétroliers associés.

Selon cette méthode, on utilise un schéma compositionnel des hydrocarbures et des gaz non hydrocarbures générés. L'action bactérienne, à l'origine de la biodégradation des hydrocarbures, génère des gaz non hydrocarbures ($CO_2$, $H_2S$) et du méthane ($CH_4$). Elle altère plus vite les hydrocarbures saturés n et les hydrocarbures saturés iso. Ensuite les structures cycliques saturées et les aromatiques peuvent être touchés. En principe les composés soufrés et azotés (NSOs) restent inchangés, ainsi que les gaz $C_2$-$C_4$ sauf dans des conditions extrêmes. Ainsi, l'ordre à priori pour l'altération est le suivant : $C_6$-$C_{14}$ sat et aromatiques, $C_{14+}$ saturés.

Ainsi, selon l'invention, on utilise les douze classes chimiques suivantes, afin de formaliser de façon compositionnelle la biodégradation des fluides (hydrocarbures + gaz) :

| | |
|---|---|
| Classe 1 | $CO_2$ |
| Classe 2 | $H_2S$ |
| Classe 3 | $C_1$ |
| Classe 4 | $C_2$-$C_4$ |
| Classe 5 | $C_6$-$C_{14}$ saturés n et $C_6$-$C_{14}$ saturés iso |
| Classe 6 | $C_6$-$C_{14}$ saturés cyclo |
| Classe 7 | $C_6$-$C_{14}$ aromatiques |
| Classe 8 | $C_{14+}$ saturés n |

(suite)

| | |
|---|---|
| Classe 9 | $C_{14+}$ saturés iso |
| Classe 10 | $C_{14+}$ saturés cyclo |
| Classe 11 | $C_{14+}$ aromatiques |
| Classe 12 | NSOs |

Avec la nomenclature classique suivante :

$C_1$ : famille d'hydrocarbures à 1 atome de carbone

$C_2$ : famille d'hydrocarbures à 2 atomes de carbone

$C_3$ : famille d'hydrocarbures à 3 atomes de carbone

$C_4$ : famille d'hydrocarbures à 4 atomes de carbone

$C_6$-$C_{14}$ ou $C_{14-}$ : hydrocarbures ayant entre 6 et 14 atomes de carbone

$C_{14+}$ : hydrocarbures ayant plus de 14 atomes de carbone

NSOs : composés hétéroatomiques généralement de haute masse moléculaires (résines et asphaltènes) contenant des fonctions azotées, soufrées et oxygénées)

Les classes 3 à 12 permettent de décrire les hydrocarbures avant et après la biodégradation. Les classes 1 et 2 permettent de prendre en compte la génération de gaz non hydrocarbure pendant la biodégradation, ce qui est important pour déterminer les condition d'exploitation d'un champ.

2- Estimation des vitesses de déplacement des fluides et de la température

Selon l'invention, on prend en compte le fait que la biodégradation n'a pas lieu uniquement au niveau de l'interface entre les hydrocarbures et l'eau au sein du réservoir pétrolier, mais dans tout le milieu poreux constituant le bassin et traversé par les hydrocarbures.

Pour ce faire, on estime la vitesse de déplacement des hydrocarbures et de l'eau présente dans le milieu poreux. A l'échelle d'une maille de simulation d'écoulement dans un modèle de bassin, on calcule en pratique une variation de saturation moyenne en hydrocarbure, notée $\Delta$SatHC. On définit aussi une saturation en eau disponible pour la réaction de biodégradation, notée $SatIR_{WBIO}$. Cette saturation est fonction du type de lithologie. Elle est faible pour des argiles, et est maximum dans des milieux poreux sphériques (type sable).

Connaissant, sur un pas de temps donné, la variation de saturation moyenne, on peut déterminer les vitesses de déplacement des fluides.

Le simulateur de bassin fournit également, de façon connue des spécialistes, une estimation de la température à chaque pas de temps et dans toutes mailles.

3- Estimation de vitesses de biodégradations

3a- Schéma réactionnel de biodégradation

L'unité de base pour les calculs de la biodégradation est la concentration molaire $C_{mi}$ des composés (classes chimiques) i pris en compte dans le schéma compositionnel. Cette concentration molaire est définie par la formule présentée dans l'équation (1).

$$C_{mi} = \frac{C_i / M_i}{C_o / M_o} \qquad (1)$$

avec

$C_i$ : concentration massique du composé i

$M_i$ : masse molaire du composé i

$C_o$ : concentration massique de l'huile

$M_o$ : masse molaire de l'huile

La masse molaire de l'huile est un paramètre qui dépend des caractéristiques du pétrole. Sa valeur est fonction du type de kérogène ayant entraîné la genèse de l'huile, de l'histoire thermique du système pétrolier et en particulier du craquage secondaire que peut avoir subit le pétrole. La masse molaire de l'huile dépend également de la biodégradation, ainsi une huile biodégradée est elle plus lourde qu'elle ne l'était avant biodégradation.

$$M_o = \text{f(Type I, II, III), f(histoire thermique), f(biodégradation)}$$

La minéralisation des hydrocarbures sous l'action de bactéries hydrocarbonoclastes conduit d'une part, à la disparition complète des hydrocarbures initialement présents et d'autre part, à la production des métabolites finaux suivants :

- $CO_2$ et $H_2O$ en conditions aérobies
- $CO_2$, $H_2O$ et $N_2$ en conditions dénitrifiantes
- $CO_2$, $H_2O$ et $H_2S$ en conditions sulfatoréductrices
- $CO_2$, et $CH_4$ en condition méthanogènes.

Ces équations décrivent les voies de biodégradation des hydrocarbures avec différents accepteurs d'électrons. Il s'agit de conditions de biodégradation aérobie (en présence d'oxygène), dénitrifiantes (en présence de nitrate), sulfatoréductrices (en présence de sulfate) et méthanogènes. Cette succession de mécanismes faisant appel à des accepteurs d'électrons ayant un potentiel d'oxydoréduction de plus en plus faible, ne peut se faire qu'après épuisement de l'accepteur d'électron précédent.

Les équations séquentielles de biodégradation d'un hydrocarbure de formule CxHy avec les principaux accepteurs d'électrons ($O_2$, $NO_3$, $SO_4$ et $H_2O$), sont présentées ci-après.

**accepteur d'électrons : $O_2$. biodégradation aérobie**

<u>Équation globale</u> : $C_xH_y + (x+1/4y)\ O_2 \xrightarrow{V_{O_2}} x\ CO_2 + 1/2y\ H_2O$

$$\underline{\text{Exemple}} : C_{16}H_{34} + (49/2)\ O_2 \xrightarrow{V_{O_2}} 16\ CO_2 + 17\ H_2O$$

**accepteur d'électrons : NO3, dénitrification**

$$\underline{\text{Équation globale}} : C_xH_y + (2/3x+1/6y)\ NO_3 \xrightarrow{V_{NO_3^{2-}}} x\ CO_2 + (1/3x+1/12y)\ N_2 + 1/2y\ H_2O$$

$$\underline{\text{Exemple}} : C_{16}H_{34} + (49/3)\ NO_3 \xrightarrow{V_{NO_3^{2-}}} 16\ CO_2 + (49/6)\ N_2 + 17\ H_2O$$

**accepteur d'électrons : SO4, sulfatoréduction**

$$\underline{\text{Équation globale}} : C_xH_y + (2/5x+1/10y)\ SO_4 \xrightarrow{V_{SO_4^{2-}}} x\ CO_2 + (2/5x+1/10y)\ H_2S + (-2/5x+2/5y)\ H_2O$$

$$\underline{\text{Exemple}} : C_{16}H_{34} + (98/10)\ SO_4 \xrightarrow{V_{SO_4^{2-}}} 16\ CO_2 + (98/10)\ H_2S + (36/5)\ H_2O$$

**accepteur d'électrons : H2O, méthanogénèse**

$$\underline{\text{Équation globale}} : C_xH_y + (x-1/4y)\ H_2O \xrightarrow{V_{H_2O}} (1/2x-1/8y)\ CO_2 + (1/2x+1/8y)\ CH_4$$

$$\underline{\text{Exemple}} : C_{16}H_{34} + (15/2)\ H_2O \xrightarrow{V_{H_2O}} (15/4)\ CO_2 + (49/4)\ CH_4$$

Ainsi, le schéma réactionnel de biodégradation selon l'invention, est défini par l'ensemble des réactions chimiques suivantes :

- pour chaque classe chimique présentes dans les hydrocarbures, les réactions avec les accepteurs d'électrons (oxygène, nitrate, sulfate ou $H_2O$) sont séquentielles : chaque classe réagit avec l'accepteur d'électrons ayant le potentiel d'oxydoréduction le plus fort, jusqu'à épuisement de cet accepteur, puis réagit avec l'accepteur d'électrons restant dans le milieu et ayant le potentiel d'oxydoréduction le plus fort. Ce mécanisme peut se répéter jusqu'à épuisement des accepteurs d'électrons.
- les classes chimiques réagissent en parallèle : il n'existe pas de mécanisme séquentiel, dans lequel une classe chimique réagirait une fois qu'une autre classe aurait fini de réagir. En fait, toutes les classes peuvent réagir en même temps, mais avec des cinétiques différentes.

3b- Expressions des vitesses de biodégradation

L'équation de vitesse de biodégradation peut s'écrire ainsi :

$$V_i = -\frac{\partial C_{mi}}{\partial t} = -\frac{C_{mi}(t_n) - C_{mi}(t_{n-1})}{t_n - t_{n-1}}$$

avec

$C_{mi}$ : concentration molaire du composé i qui fait l'objet de la biodégradation

$C_{mi}(t_n)$ : concentration molaire du composé i au temps $t_n$

$C_{mi}(t_{n-1})$ : concentration molaire du composé i au temps $t_{n-1}$

Cette vitesse $V_i$ est entre autre fonction de l'accepteur d'électron, de la biomasse, du composé *i*, de la température T, de l'hydrodynamique des fluides et de la diffusion.

Les accepteurs d'électron sont : $O_2$, $NO_3$, $SO_4$, $H_2O$. Ce paramètre dépend du type de réservoir. La biomasse concerne les éléments nutritifs (N, P, K) et les éléments traces (Fe, Mg, Co...). Ce paramètre dépend également du type de réservoir. Le composé i est une des classes chimiques définies dans le schéma compositionnel de l'étape 1. La température T intervient à un instant donné (température au moment de la réaction), mais également en fonction de l'histoire thermique (paléostérilisation). L'hydrodynamique concerne le déplacement de l'huile dans le réservoir et/ou dans les chemins de migration, ainsi que l'évolution du contact huile/eau, et du flux d'eau.

3c- Détermination des vitesses globales de biodégradation

Selon l'invention, on utilise préférentiellement les réactions globales. On affecte à chacune d'elle une vitesse, selon la classe chimique et l'accepteur d'électrons. Ces vitesses de réactions, sont appelées vitesses globales.

Ainsi, on détermine les vitesses globales de chacune des classes chimiques du schéma compositionnel, en fonction d'une part de l'accessibilité des hydrocarbures aux bactéries, c'est à dire leur concentration potentiellement soluble dans l'eau,et d'autre part, de leur biodégradabilité intrinsèque (capacité des microorganismes à les dégrader).

On considère qu'une telle vitesse globale est le produit de trois termes : un premier terme ($R_t$) qui permet de prendre en compte l'effet du temps de séjour dans le milieu poreux, un second terme ($R_T$) pour prendre en compte l'effet de la température, et un troisième terme ($K_C$) pour prendre en compte les spécificités de chacune des classes chimiques.

Le premier terme ($R_t$) permettant de prendre en compte l'effet du temps de séjour dans le milieu poreux représente un rendement de biodégradation. Il peut être déterminé à partir de la courbe représentés sur la figure 3, qui illustre l'évolution du rendement de biodégradation $R_t$ (%) en fonction du temps *t* (en année). Dans cette figure on définit un temps plateau (ici de 2.000 ans) au delà duquel le rendement est supérieur à 95 % et en de ça duquel le rendement sera d'autant plus faible que le temps diminue.

Le second terme ($R_T$) permettant de prendre en compte l'effet de la température représente un rendement global de biodégradation. Il peut être déterminé en considérant que le rendement global $R_T$ évolue en fonction de la température selon une courbe gaussienne. Selon un exemple, illustré sur la figure 4, cette courbe gaussienne est centrée sur une température d'environ 30°C et s'annule pour 0°C et environ 70°C.

Le troisième terme ($V_C$) permettant de prendre en compte les spécificités de chacune des classes chimiques est composé de deux termes. Un premier rend compte de la biodégradabilité intrinsèque des différentes classes de composés. Si $V_{bio}$ est la vitesse globale maximum de biodégradabilité, on peut classer la biodégradabilité des différentes classes chimiques d'hydrocarbures comme suit :

$$(V_{bio})_{C14\text{-}sat} = (V_{bio})_{max}$$

$$(V_{bio})_{C14\text{-}aro} = 0.6 \times (V_{bio})_{max}$$

$$(V_{bio})_{C14+n} = (V_{bio})_{max}$$

$$(V_{bio})_{C14+iso} = (0.8) \times (V_{bio})_{max}$$

$$(V_{bio})_{C14+cyclanes} = (0.3) \times (V_{bio})_{max}$$

$$(V_{bio})_{C14+aro} = (0.1) \times (V_{bio})_{max}$$

$$(V_{bio})_{C14+nso} = (0.0) \times (V_{bio})_{max}$$

Le second terme permettant de définir $V_C$ rend compte de l'accessibilité des hydrocarbures dans le milieu poreux, c'est à dire leur capacité à être accessibles aux bactéries (par exemple les aromatiques $C_6$-$C_{14}$ sont les hydrocarbures les plus solubles dans l'eau, c'est donc eux qui auront l'accessibilité maximale).

$$(V_{accès})_{C14-aro} = (V_{accès})_{max}$$

$$(V_{accès})_{C14-sat} = 0.4 \times (V_{accès})_{max}$$

$$(V_{accès})_{C14+chains} = (0.2) \times (V_{accès})_{max}$$

$$(V_{accès})_{C14+cyclanes} = (0.2) \times (V_{accès})_{max}$$

$$(V_{accès})_{C14+aro} = (0.3) \times (V_{accès})_{max}$$

$$(V_{accès})_{C14+nso} = (0.0) \times (V_{accès})_{max}$$

Ainsi, le second terme $V_C$ pour les différentes classes chimiques est :

$$V_C\ C_{14}\text{-sat} = (V_{bio})max \quad \times \quad 0.4 \times (V_{accès})max$$

$$V_C\ C_{14-}aro = 0.3 \times (V_{bio})max \times (V_{accès})max$$

$$V_C\ C_{14+}n = (V_{bio})max \times 0.3) \times (V_{accès})max$$

$$V_C\ C_{14+}iso = 0.7 \times (V_{bio})max \times 0.2x\ (V_{accès})max$$

$$V_C\ C_{14+}cyclanes = 0.4 \times (V_{bio})max \times 0.2 \times (V_{accès})max$$

$$V_C\ C_{14+}aro = 0.15 \times (V_{bio})max \times 0.3 \times (V_{accès})max$$

$$V_C\ NSO = 0$$

Ainsi, en prenant en compte l'ensemble des termes ($V_{max}$ = $(V_{bio})$max x $(V_{accès})$max), les vitesses globales (V) de biodégradation pour les différentes classes chimiques sont donc les suivantes

$$V\ C_{14-}sat = (V_{bio})max \times 0.4 \times (V_{accès})max = 0.4xV_{max}$$

$$V\ C_{14-}aro = 0.3 \times (V_{bio})max \times (V_{accès})max = 0.3xV_{max}$$

$$V\ C_{14+}n = (V_{bio})max \times 0.3) \times (V_{accès})max = 0.2xV_{max}$$

$$V\ C_{14+}iso = (0.7) \times (V_{bio})max \times 0.2x\ (V_{accès})max = 0.18xV_{max}$$

$$V\ C_{14+}cyclanes = (0.4) \times (V_{bio})max \times (0.2) \times (V_{accès})max = 0.08xV_{max}$$

$$V\ C_{14+}aro = (0.15) \times (V_{bio})max \times (0.3) \times (V_{accès})max = 0.05xV_{max}$$

$$V\ NSO = 0$$

4- Détermination de la variation compositionnelle des hydrocarbures.

On détermine à chaque pas de temps la variation de concentration ΔCi d'une classe chimique i au cours de l'intervalle de temps Δt, au moyen de la formule suivante :

si la température T est supérieure à une température au dessus de laquelle il n'y a plus d'activité bactérienne, ΔCi = - Vi(Δt, T)* $\Delta Sat_{HC}$* $SatIR_{WBIO}$* Ci (tn)*Δt, avec $\Delta t = t_{n+1} - t_n$.

Ainsi la méthode permet de déterminer la composition de fluides présents dans un milieu poreux suite à une biodégradation. Ces fluides correspondent d'une part aux hydrocarbures biodégradés, et d'autres part aux métabolites produits lors de cette biodégradation, et notamment les gaz d'origine biologique associés : $CH_4$, $CO_2$ et $H_2S$.

## Revendications

1. Méthode mise en oeuvre par ordinateur pour déterminer une composition d'hydrocarbures présents dans un milieu poreux d'un bassin sédimentaire, dans laquelle on modélise une biodégradation subie par lesdits hydrocarbures, **caractérisé en ce que** l'on estime des vitesses de déplacement desdits hydrocarbures et des vitesses de déplacement de l'eau présente dans ledit milieu poreux, depuis un instant de genèse dans une roche mère jusqu'à une accumulation éventuelle dans une roche réservoir, et on modélise la biodégradation dans l'ensemble dudit milieu poreux en fonction du temps, depuis la genèse dans la roche mère, en considérant que ladite composition d'hydrocarbures varie proportionnellement auxdites vitesses de déplacement.

2. Méthode selon la revendication 1, dans laquelle la modélisation comporte les étapes suivantes :

   - on discrétise ledit milieu poreux dudit bassin sédimentaire en un ensemble de mailles ;
   - on définit un schéma compositionnel de biodégradation à partir d'au moins les classes chimiques suivantes : $CO_2$, $H_2S$, $C_1$, $C_2$-$C_4$, $C_6$-$C_{14}$ saturés n et $C_6$-$C_{14}$ saturés iso, $C_6$-$C_{14}$ saturés cyclo, $C_6$-$C_{14}$ aromatiques, $C_{14+}$ saturés n, $C_{14+}$ saturés iso, $C_{14+}$ saturés cyclo, $C_{14+}$ aromatiques, NSOs ;
   - on détermine, à l'instant de genèse des hydrocarbures, une quantité $C_i$ de chacune desdites classes chimiques contenues dans lesdits d'hydrocarbures ;
   - on définit un pas de temps Δt, discrétisant le temps à partir de la genèse des hydrocarbures, et pour chaque maille et chaque pas de temps Δt :
   - on estime lesdites vitesses de déplacement en déterminant une variation de saturation moyenne en hydrocarbure $\Delta Sat_{HC}$ et une saturation en eau disponible pour la réaction de biodégradation $Sat_{WBIO}$;
   - on détermine une température T du milieu poreux ;
   - on détermine des vitesses de biodégradations $V_i$ pour chacune desdites classes chimiques, en fonction de la concentration potentielle des classes chimiques dans l'eau, de leur biodégradabilité intrinsèque, de leur réactivité par rapport auxdits accepteurs d'électrons, de la température du milieu, et du temps de séjour desdits hydrocarbures au sein du milieu ; et
   - on détermine une variation de concentration $\Delta C_i$ d'une classe chimique i au cours de l'intervalle de temps Δt, à partir desdites quantités d'hydrocarbures, de $\Delta Sat_{HC}$, de $Sat_{WBIO}$, et de T, et desdites vitesses de biodégradation.

3. Méthode selon la revendication 2, dans laquelle on détermine la variation de concentration $\Delta C_i$ au moyen de la formule suivante :

$$\Delta C_i = - V_i(\Delta t, T)^* \Delta Sat_{HC}{}^* Sat_{WBIO}{}^* Ci\ (t_n)^* \Delta t * I_{STERIL},$$

   avec :

$$\Delta t = t_{n+1} - t_n.$$

   $I_{STERIL}$ = 1 si la température T n'est jamais supérieure à une température de pasteurisation,

$I_{STERIL}$ = 0 sinon.

**4.** Méthode selon l'une des revendications précédentes, dans laquelle lesdites vitesses de réactions sont définies par un produit d'un premier terme ($R_t$) qui permet de prendre en compte un effet du temps de séjour dans le milieu poreux, un second terme ($R_T$) pour prendre en compte un effet de la température, et un troisième terme ($K_C$) pour prendre en compte l'accessibilité et la biodégradabilité intrinsèque de chacune des classes chimiques.

**5.** Méthode selon la revendication 4, dans laquelle le premier terme $R_t$ représente un rendement de biodégradation et est déterminé au moyen d'une première courbe d'évolution dudit rendement $R_t$ en fonction du temps de séjour dans le milieu.

**6.** Méthode selon la revendication 5, dans laquelle ladite première courbe est une fonction exponentielle du temps, où ledit rendement $R_t$ est supérieur à 95 % au-delà d'environ 2000 ans.

**7.** Méthode selon l'une des revendications 4 à 6, dans laquelle le second terme $R_T$ représente un rendement de biodégradation et est déterminé au moyen d'une courbe gaussienne d'évolution dudit rendement $R_T$ en fonction de la température dans le milieu.

**8.** Méthode selon la revendication 7, dans laquelle ladite courbe gaussienne est centrée sur une température d'environ 30°C et s'annule pour 0°C et environ 70°C.

**9.** Méthode selon l'une des revendications 4 à 8, dans laquelle le troisième terme ($V_C$) est déterminé en considérant que la vitesse de biodégradabilité intrinsèque des $C_6$-$C_{14}$ saturés est plus grande que celles des autres classes, et en considérant que la vitesse d'accessibilité maximale des $C_6$-$C_{14}$ aromatiques est plus grande que celles des autres classes.

**10.** Méthode selon l'une des revendications 4 à 9, dans laquelle lesdites vitesses de réactions sont définies par :

$$V\ C_{14-}sat = 0.4xV_{max}$$

$$V\ C_{14-}aro = 0.3xV_{max}$$

$$V\ C_{14+}n = 0.2xV_{max}$$

$$V\ C_{14+}iso = 0.18xV_{max}$$

$$V\ C_{14+}cyclanes = 0.08xV_{max}$$

$$V\ C_{14+}aro = 0.05xV_{max}$$

$$V\ NSO = 0$$

où $V_{max}$ est vitesse maximale de biodégradation définie par le produit de la vitesse maximale de biodégradabilité intrinsèque par la vitesse maximale d'accessibilité.

**11.** Méthode selon l'une des revendications précédentes, dans laquelle on détermine également une quantité de gaz acides produit lors de la biodégradation, pour déterminer des conditions d'exploitation d'une roche réservoir dudit bassin.

**12.** Méthode selon l'une des revendications précédentes, dans laquelle on détermine également une quantité de méthane produit lors de la biodégradation, pour déterminer des conditions d'exploitation d'une roche réservoir dudit bassin.

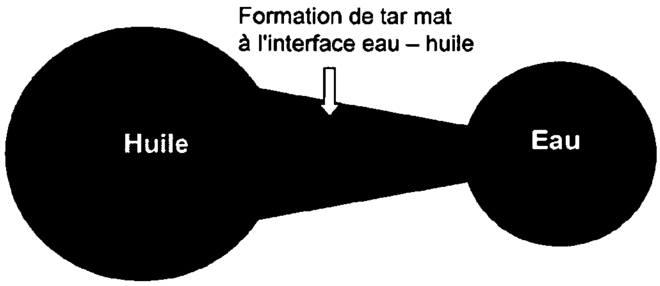
Formation de tar mat
à l'interface eau – huile
Huile
Eau

Fig. 1

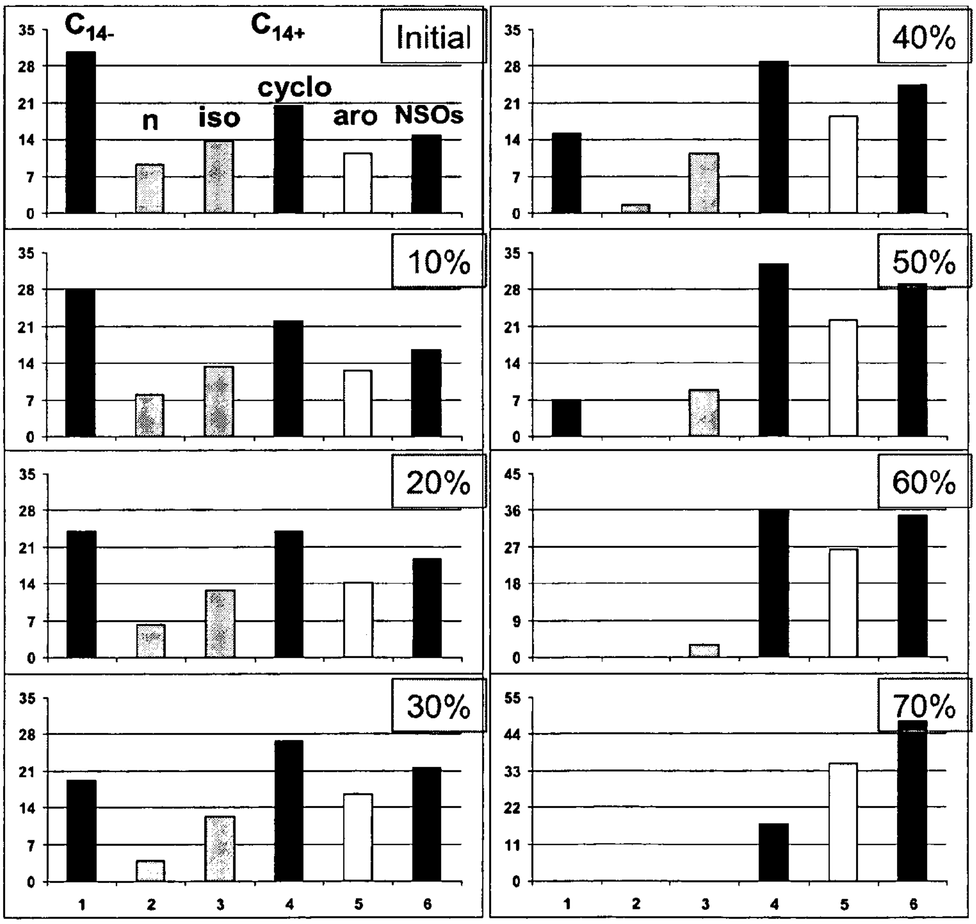
C14-
C14+
Initial
n
iso
cyclo
aro
NSOs
10%
20%
30%
40%
50%
60%
70%

Fig. 2

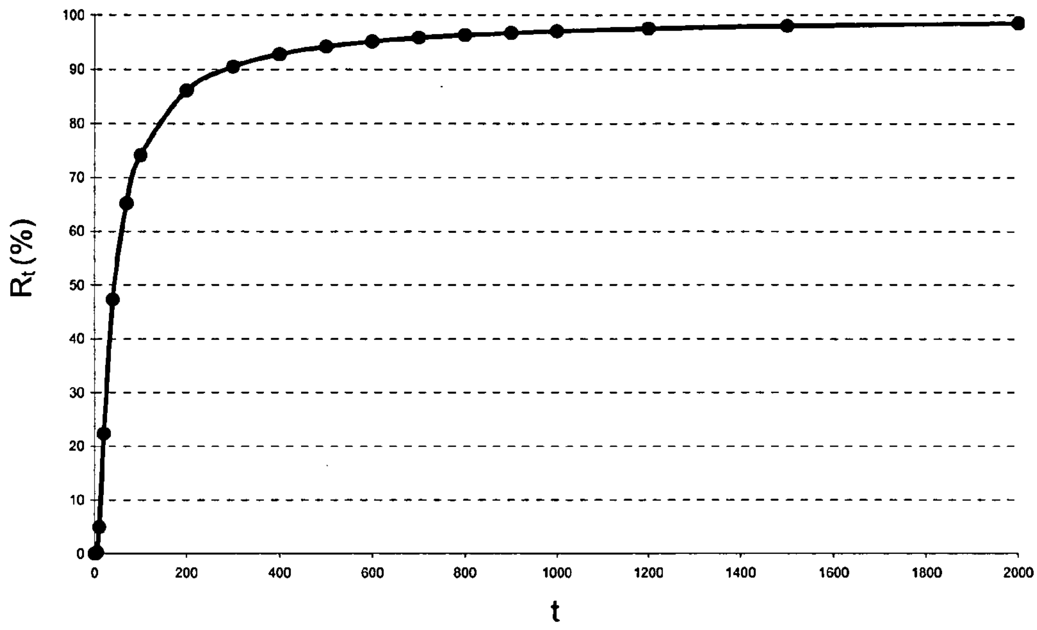
$R_t$ (%)
100
90
80
70
60
50
40
30
20
10
0
0
200
400
600
800
1000
1200
1400
1600
1800
2000
t

Fig. 3

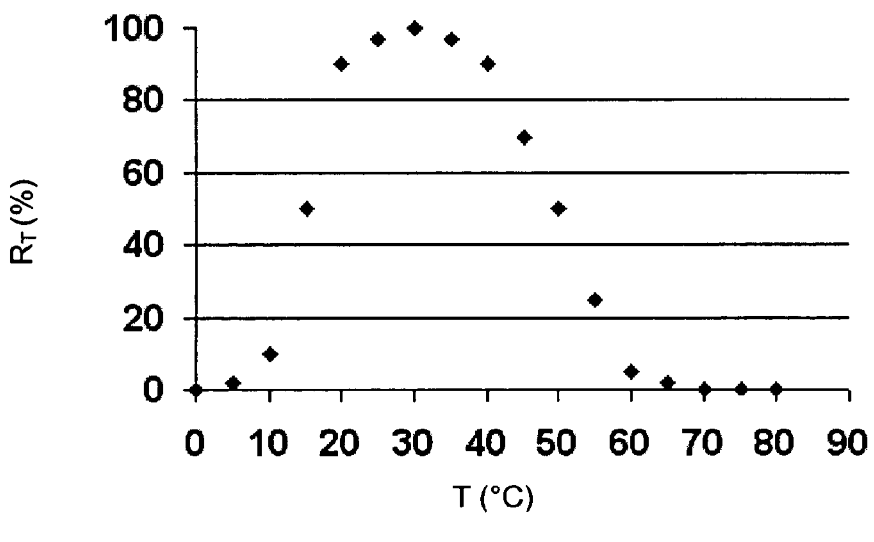
$R_T$ (%)
100
80
60
40
20
0
0 10 20 30 40 50 60 70 80 90
T (°C)

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1436412 A
- FR 2906482


### Littérature non-brevet citée dans la description


- **Conan et al.** Advances in Organic Geochemistry. Pergamon Press, 1979, 1-17
- **Peters ; Moldowan.** The biomarker guide. Prentice Hall, 1993
- **Larter et al.** *Organic Geochemistry,* 2003, vol. 4, 6001-613
- **Behar et al.** *Organic Geochemistry,* 2006, vol. 37, 1042-1051
- **Barros Penteado et al.** *Organic Geochemistry,* 2007, vol. 38, 1197-1211
- **Wilhems A ; Larter SR ; Head I ; Farrimond P ; di Primio R ; Zwach C.** Biodegradation of oil in uplifted basins prevented by deep-burial sterilization. *Nature,* 2001, vol. 411, 1034-1037